# EUROPEAN PATENT APPLICATION

(11) **EP 4 400 630 A1**
(43) Date of publication of application: **17.07.2024**
(21) Application number: 22866531.1
(22) Date of filing: 05.09.2022
(51) Int. Cl.: C25B 1/04, C25B 15/023, A61M 16/16, B01F 23/237

(54) **HYDROGEN WATER GENERATING DEVICE WITH WARNING FUNCTION, AND HYDROGEN PRODUCTION SYSTEM THEREOF**

(30) Priority: 08.09.2021 CN 202122167078 U
(71) Applicant: Lin, Hsin-Yung, Taoyuan City, Taiwan 33341 (CN)
(72) Inventor: Lin, Hsin-Yung, Taoyuan City, Taiwan 33341 (CN)
(74) Representative: Wang, Bo
(86) International application number: PCT/CN2022/116944
(87) International publication number: WO 2023/036068

(57) **Abstract**

A hydrogen water generator with warning function includes a container, a gas input port, a handle, a diffusing pipe, a gas output port, and a water port. The gas input port receives gas comprising hydrogen. The diffusing pipe is configured in the containing space and coupled to the gas input port. The gas output port receives the gas comprising hydrogen in the containing space and outputs it to the outside. The water port outputs the water to the outside. The gas comprising hydrogen enters into the water in the containing space to generate hydrogen water and humidified gas comprising hydrogen to be outputted to the outside of the container through the water port and the gas output port respectively. The container emits a first color light when the system is under a normal condition, but emits a second color light when the system is under an abnormal condition.

## Description

### BACKGROUND OF THE INVENTION

### 1. Field of the invention

The present invention relates to a hydrogen water generator and a hydrogen gas generating system that can generate the hydrogen water and the humidified gas, and more particularly, to a hydrogen water generator and a hydrogen gas generating system that can generate the hydrogen water and the humidified gas and has warning function simultaneously.

### 2. Description of the prior art

As people have always been paying much attention on health developments, many developments in medical technology are often targeted on treating diseases and prolonging human life. Most of the treatments in the past are passive, which means that the disease is treated only when it occurs. The treatments include operations, medication treatments, radiation therapies, or even medical treatments for cancer. However, in recent years, most of the researches from medical experts are gradually moving towards preventive medical methods, such as research on healthy food, screening and the prevention of inherited diseases, which actively prevents diseases from occurring in the future. Due to the focus of the prolongation of human life, many anti-aging and anti-oxidation technologies including skin care products and anti-oxidation food/medicine are gradually being developed and have becoming increasingly popular to the general public.

Free radicals are molecules or ions of one or more unpaired electrons that are quite active and tend to pull nearby electrons into them for stable. The normal metabolism of the human body is an oxidative reaction that naturally produces the free radicals. Moreover, in order to maintain the normal functioning of the human body, it is necessary to produce many useful chemical substances or resist foreign germs, such as the production of enzymes or the phagocytosis of germs by leukocytes, etc. At this time, the free radicals are also generated. In addition to the above internal factors, the human body will also generate additional free radicals due to various external environmental influences, such as smoking, alcohol abuse and other bad habits, radiation, ultraviolet and electromagnetic waves, and even various environmental pollutions. Because the oxidative power of the free radicals is quite strong, they will attack and damage cells. According to current research, the free radicals are the main cause of various chronic diseases, cancer, and even aging.

The above-mentioned free radicals can synthesize water with inhaled hydrogen gas and to be excreted from the body because hydrogen is an antioxidant substance with strong reducing power. Using hydrogen gas to reduce the number of free radicals in the human body can achieve the function of restoring an acidic body to a healthy alkaline body, thereby achieving anti-oxidation, anti-aging, and even eliminating chronic diseases and beauty care effects. There are even clinical experiments showing that for some bedridden patients having lung damage caused by long-term breathing of high concentrations of oxygen can relieve the symptoms of lung damage by inhaling hydrogen gas. Since the structure of the human trachea is not suitable for inhaling too dry gas, the hydrogen gas must be properly humidified before it can be inhaled by patients.

In addition to the inhalation of humidified hydrogen gas, the effect of preventing or treating various diseases can also be achieved by drinking or injecting hydrogen-rich water (hydrogen gas water or hydrogen water). However, the hydrogen gas has low solubility in water, and the human body needs to drink or inject a larger amount of hydrogen water to achieve a better therapeutic effect. Therefore, how to manufacture the humidified hydrogen gas and hydrogen water efficiently has become an urgent goal to be achieved in the art.

The humidified hydrogen gas and hydrogen water can remove free radicals and prevent diseases or anti-aging. In addition to the humidified hydrogen gas and hydrogen water can be used in hospitals or similar medical units, they can further achieve the effect of early prevention of diseases when used in home life. In the prior art, the hydrogen water generating device used at home electrolyzes water or reacts with magnesium to make the water contain hydrogen gas. However, the way of electrolyzing water will deposit minerals on the electrodes and affect the water quality, and the magnesium oxide will remain in the water by magnesium reaction. Both of the ways must process the filtering process to ensure the quality of the hydrogen water. However, the additional filtering process will cause part of the hydrogen gas in the hydrogen water to escape to the external environment and reduce its effect. Therefore, it is necessary to develop a hydrogen water generator with simple procedures and no excess residues or minerals for domestic uses.

In addition, when the above-mentioned hydrogen water generator adds the gas comprising hydrogen to water, and an abnormal condition occurs in the device to cause the gas comprising hydrogen leakage, which might cause an unexpected accident. Therefore, it is also necessary to warn the user for the above-mentioned abnormal conditions to avoid accidents.

### SUMMARY OF THE INVENTION

The present invention provides a hydrogen water generator and a hydrogen gas generating system with warning function, which have a simple structure and easy to operate. The device and system can effectively generate hydrogen water and humidified gas and provide hydrogen water generation without excessive residues or minerals, can also provide intuitive and effective warning function, making it more practical.

The present invention provides a hydrogen water generator with warning function including a container, a handle, a gas input port, a diffusing pipe, a gas output port, a water port and a luminous component. The container includes a case having an opening and a cover disposed on the opening. The case forms a containing space to contain water. The handle is disposed on the case of the container for a user to hold. The gas input port is disposed on the container to receive a gas comprising hydrogen from the outside of the container. The diffusing pipe is disposed in the containing space and communicated with the gas input port to receive the gas comprising hydrogen. A part of the diffusing pipe is disposed in the water of the containing space, and the pipe wall of the diffusing pipe has a plurality of holes. The gas comprising hydrogen passes from the diffusing pipe into the water of the containing space through the holes to generate a hydrogen water and a humidified gas comprising hydrogen. The gas output port is disposed on the container to receive the humidified gas comprising hydrogen from the containing space and output the humidified gas comprising hydrogen to the outside of the container. The water port is disposed on the container to output the hydrogen water of the containing space to the outside of the container. The luminous component is configured to emit light toward the hydrogen water generator from the outside of the hydrogen water generator, and the emitted light passes through the container to emit light from the container. The luminous component is a LED for showing the first color light under the normal state of the hydrogen water generator and showing the second color light under the abnormal state of the hydrogen water generator.

Wherein, the container has a transparent outer wall. The luminous component is configured on the container to emit light toward the inside of the hydrogen water generator, and the emitted light is emitted from the container through the transparent outer wall.

Wherein, the gas input port, the gas output port and the water port are disposed on the cover.

Wherein, the hydrogen water generator further includes a water cover detachably covering on the water port. The water cover includes a cover sheet and a connecting strip.

Wherein, the diffusing pipe further includes a plurality of micro filters corresponding to the holes respectively. The micro filters are configured to filter the gas comprising hydrogen passing through the holes, and the gas comprising hydrogen forms a plurality of micro gas bubbles after entering into the water of the containing space through the holes.

Wherein, the hydrogen water generator further includes a hydrogen concentration detecting device configured to detect whether a hydrogen concentration of the outside of the container is higher than a preset hydrogen concentration. The container shows the first color light when the hydrogen concentration is lower than the preset hydrogen concentration, and the container shows the second color light when the hydrogen concentration is higher than the preset hydrogen concentration.

The present invention also provides a hydrogen gas generating system including a hydrogen gas generating device, a hydrogen water generator, a hydrogen gas detecting device and a warning device. The hydrogen water generator includes a container, a handle, a gas input port, a diffusing pipe, a gas output port and a water port. The hydrogen gas generating device is configured to generate a gas comprising hydrogen. The container of the hydrogen water generator is detachably disposed on the hydrogen gas generating device. The hydrogen water generator includes a case having an opening and a cover configured on the opening, and the case forms a containing space to contain water. The handle is disposed on the case of the container for a user to hold. The gas input port is disposed on the container to receive a gas comprising hydrogen from the hydrogen gas generating device. The diffusing pipe is disposed in the containing space and communicated with the gas input port to receive the gas comprising hydrogen. A part of the diffusing pipe is disposed in the water of the containing space, and the pipe wall of the diffusing pipe has a plurality of holes. The gas comprising hydrogen passes from the diffusing pipe into the water of the containing space through the holes to generate a hydrogen water and a humidified gas comprising hydrogen. The gas output port is disposed on the container to receive the humidified gas comprising hydrogen from the containing space and output the humidified gas comprising hydrogen to the outside of the container. The water port is disposed on the container to output the hydrogen water of the containing space to the outside of the container. The hydrogen gas detecting device is configured to detect whether a hydrogen concentration around the hydrogen gas generating device is higher than a preset hydrogen concentration. The warning device is coupled to the hydrogen gas detecting device and configured to generate a warning signal when the hydrogen concentration detected by the hydrogen gas detecting device is higher than the preset hydrogen concentration.

Wherein, the warning device is a LED luminous component disposed on the hydrogen gas generating device. The LED luminous component causes the container to show a first color light when the hydrogen concentration detected by the hydrogen gas detecting device is lower than the preset hydrogen concentration. The LED luminous component generates the warning signal to cause the container to show a second color light when the hydrogen concentration detected by the hydrogen gas detecting device is higher than the preset hydrogen concentration.

Wherein, the warning device is a LED luminous component disposed on the hydrogen water generator. The LED luminous component causes the container to show a first color light when the hydrogen concentration detected by the hydrogen gas detecting device is lower than the preset hydrogen concentration. The LED luminous component generates the warning signal to cause the container to show a second color light when the hydrogen concentration detected by the hydrogen gas detecting device is higher than the preset hydrogen concentration.

Wherein, the hydrogen gas detecting device is disposed in the hydrogen gas generating device to detect whether the hydrogen concentration in the hydrogen gas generating device is higher than the preset hydrogen concentration. The warning device generates the warning signal to cause the container to show a second color light when the hydrogen concentration detected by the hydrogen gas detecting device is higher than the preset hydrogen concentration.

Wherein, the hydrogen gas detecting device is disposed outside of the hydrogen gas generating device, and the hydrogen gas detecting device is coupled to the hydrogen gas generating device by wireless transmission to detect whether the hydrogen concentration around the hydrogen gas generating device is higher than the preset hydrogen concentration. The warning device generates the warning signal to cause the container to show a second color light when the hydrogen concentration detected by the hydrogen gas detecting device is higher than the preset hydrogen concentration.

Wherein, the hydrogen gas generating system further includes a plurality of associated hydrogen gas generating devices. Each of associated hydrogen gas generating devices includes an associated container and an associated warning device. The associated warning device is disposed on a case or the associated container of the associated hydrogen gas generating device. The hydrogen gas detecting device is coupled to the associated hydrogen gas generating devices by wireless transmission. When the hydrogen concentration around the hydrogen gas generating device or the hydrogen concentration around the associated hydrogen gas generating devices detected by the hydrogen gas detecting device is higher than the preset hydrogen concentration, the warning device and the associated warning devices cause the container and the associated containers to show a second color light respectively.

Wherein, the warning device is a sound generating component configured to generate a warning sound as the warning signal when the hydrogen concentration detected by the hydrogen gas detecting device is higher than the preset hydrogen concentration.

Wherein, the hydrogen gas generating device further includes a capacitive water level meter configured to detect the water level of the water contained in the container of the hydrogen water generator when the container is configured on the hydrogen gas generating device.

Wherein, the hydrogen gas generating device further includes a controlling module connected to the capacitive water level meter, and the capacitive water level meter includes a high water level sensor and a low water level sensor. When the container is detached from the hydrogen gas generating device to cause the high water level sensor and the low water level sensor unable to detect a high water level and a low water level of the container, the controlling module controls the hydrogen gas generating device to stop for generating the gas comprising hydrogen.

Wherein, the hydrogen gas generating device further includes a water tank, an electrolysis module, an integrated passageway module, a condensing and filtering module and a humidifying module. The water tank is configured for containing water. The electrolysis module is disposed in the water tank and configured to electrolyze the water to generate the gas comprising hydrogen. The integrated passageway module is vertically disposed above the water tank and connected to the water tank to receive the gas comprising hydrogen. The condensing and filtering module is coupled to the integrated passageway module to receive and filter the gas comprising hydrogen from the integrated passageway module. The humidifying module is vertically disposed above the water tank and located between the water tank and the integrated passageway module. The humidifying module is coupled to the integrated passageway module to receive and humidify the filtered gas comprising hydrogen.

Wherein, the condensing and filtering module has a condensing flow channel, and the condensing and filtering module is detachably disposed in the integrated passageway module to receive the gas comprising hydrogen from the integrated passageway module and filter the gas comprising hydrogen in the condensing flow channel.

Wherein, the hydrogen gas generating device further includes a nebulizer coupled to the integrated passageway module to receive the humidified gas comprising hydrogen from the integrated passageway module. The nebulizer is configured to generate an atomized gas and mix the atomized gas with the gas comprising hydrogen to form and output a health gas.

Through the above structural description, the present invention can achieve the following technical effects:
1. The device and system can quickly and easily form hydrogen water and humidified gas comprising hydrogen (or humidified gas), and the generated hydrogen water does not contain excessive minerals or magnesium oxide. Therefore, the device and system does not need to perform filtration procedures and can maintain high solubility.
2. Illuminating the micro bubbles in the container of the hydrogen water generator through the luminous component can also produce visual effects on the appearance, which can stimulate consumers' desire to buy and allow users to understand the current operating status of the system at the same time.
3. Furthermore, the device and system can detect the water level in the hydrogen water generator, can also detect whether the hydrogen water generator leaves the hydrogen generating device to stop generating the gas comprising hydrogen, further ensuring the safety of the system and users.
4. The device and system can detect whether there is leakage of gas comprising hydrogen or the environmental hydrogen concentration exceeds the standard, and then alerts the user or directly shuts down the device to avoid accidents.

The advantages and spirit of the present invention can be further understood by the following detailed description and with reference of the diagrams.

### BRIEF DESCRIPTION OF THE APPENDED DRAWINGS

FIG. 1 is a schematic diagram illustrating a hydrogen gas generating system according to an embodiment of the present invention.
FIG. 2 is a schematic diagram illustrating a hydrogen gas generating device and a hydrogen water generator of the hydrogen gas generating system of FIG. 1.
FIG. 3 is an inside structural schematic diagram illustrating the hydrogen water generator of FIG. 2.
FIG. 4 is an inside schematic diagram illustrating the hydrogen water generator according to another embodiment of the present invention.
FIG. 5 is an exploded diagram illustrating the diffusing pipe of FIG. 3.
FIG. 6 is a sectional schematic diagram illustrating the diffusing pipe of FIG. 5.
FIG. 7 is a schematic diagram illustrating the hydrogen gas generating system according to another embodiment of the present invention.
FIG. 8A is a schematic diagram illustrating an off-board hydrogen detecting component according to an embodiment of the present invention.
FIG. 8B is a schematic diagram illustrating the off-board hydrogen detecting component according to another embodiment of the present invention.
FIG. 9 is a schematic diagram illustrating the cooperation of the off-board hydrogen detecting component and a plurality of hydrogen gas generating systems according to another embodiment of the present invention.
FIG. 10A is an assembly diagram illustrating a water tank, an electrolysis module and a heat dissipater of the hydrogen gas generating device according to an embodiment of the present invention.
FIG. 10B is a sectional diagram illustrating the water tank, the electrolysis module and the heat dissipater of FIG. 10A.
FIG. 10C is an assembly diagram illustrating the water tank, the electrolysis module and the heat dissipater of the hydrogen gas generating device according to another embodiment of the present invention.
FIG. 11 is a structural schematic diagram illustrating the water tank, the heat dissipater and the fan of the hydrogen gas generating device in another one perspective.
FIG. 12 is a simple exploded diagram illustrating the hydrogen gas generating device according to an embodiment of the present invention.
FIG. 13 is a simple schematic diagram illustrating the hydrogen gas generating device in another one perspective of FIG. 12.
FIG. 14 is a simple exploded diagram illustrating the hydrogen gas generating device according to an embodiment of the present invention.

### DETAILED DESCRIPTION OF THE INVENTION

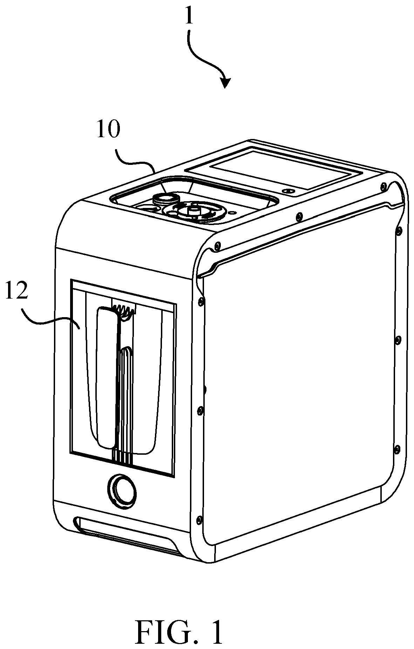

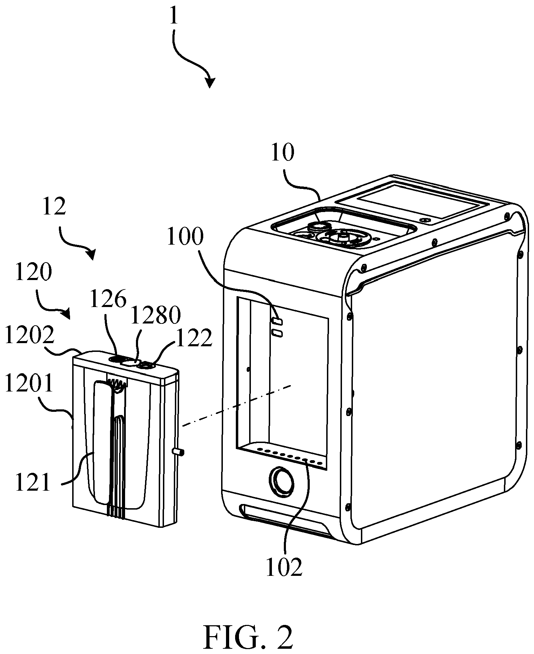

Please refer to FIG. 1 and FIG. 2. FIG. 1 is a schematic diagram illustrating a hydrogen gas generating system 1 according to an embodiment of the present invention. FIG. 2 is a schematic diagram illustrating a hydrogen gas generating device 10 and a hydrogen water generator 12 with warning function of the hydrogen gas generating system 1 of FIG. 1. As shown in FIG. 1 and FIG. 2, the hydrogen water generator 12 is detachably disposed on the hydrogen gas generating device 10. The hydrogen gas generating device 10 can generate a gas comprising hydrogen. The hydrogen gas generating device 10 can include an electrolysis module, a condensing and filtering module, an integrated passageway module, a humidifying module, an activated carbon filter module, etc., to provide the filtered gas comprising hydrogen by electrolyzing water. Alternatively, the hydrogen gas generating device 10 can also include a gas storing unit for storing the gas comprising hydrogen and provide the gas comprising hydrogen from the gas storing unit.

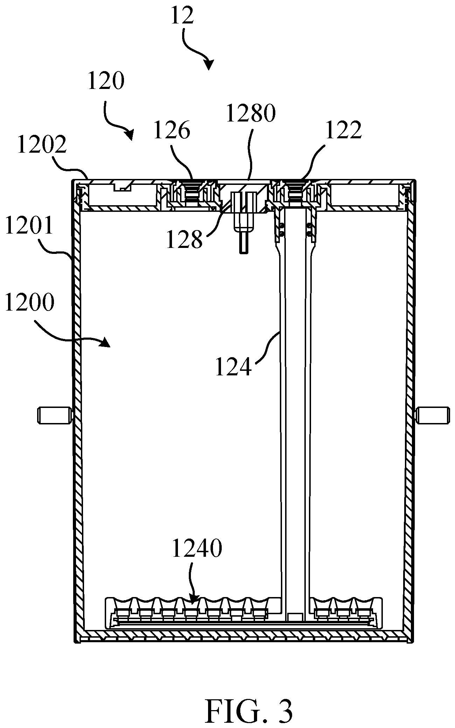

Please refer to FIG. 2 and FIG. 3. FIG. 3 is an inside structural schematic diagram illustrating the hydrogen water generator 12 of FIG. 2. As shown in FIG. 2 and FIG. 3, the hydrogen water generator 12 includes a container 120, a gas input port 122, a diffusing pipe 124, a gas output port 126 and a water port 128. The container 120 includes a case 1201 and a cover 1202. The case 1201 has an opening, the cover 1202 is disposed on the opening to cover the opening, and the case 1201 forms a containing space 1200 to contain water. The gas input port 122, the gas output port 126 and the water port 128 are disposed on the cover 1202 of the container 120 and configured to communicate with the containing space 1200 and the outer of the container 120. The diffusing pipe 124 is disposed in the containing space 1200 of the container 120. When the containing space 1200 contains the water, a part of the diffusing pipe 124 is immersed in the water. Furthermore, one side of the diffusing pipe 124 is connected to the gas input port 122, and the other side that immersed in the water of the diffusing pipe 124 includes a plurality of holes 1240.

When the hydrogen water generator 12 is connected to the hydrogen gas generating device 10, the gas comprising hydrogen generated or provided by the hydrogen gas generating device 10 can be received by the gas input port 122. Then, the gas comprising hydrogen further enters to the diffusing pipe 124 and passes the holes 1240 that immersed in the water of the diffusing pipe 124 into the water of the containing space 1200. The gas comprising hydrogen forms a plurality of micro gas bubbles in the water after entering the containing space 1200 through the holes 1240 from the inside of the diffusing pipe 124 to increase the contact area of the gas comprising hydrogen and water.

The gas comprising hydrogen forms amount of micro gas bubbles suspending in the water and moving upward after passing through the holes 1240 of the diffusing pipe 124. The gas bubbles carry the moisture to be humidified in the process of moving upward. The form of a large number of micro gas bubbles greatly increases the contact area of the gas comprising hydrogen and water, which further strengthens the degree of humidification. The humidified gas comprising hydrogen can be called humidified gas. On the other hand, the amount of micro gas bubbles formed by the gas comprising hydrogen can be effectively dispersed in the water to improve the solubility of hydrogen in the water, so that a humidified gas and a hydrogen water can be formed or generated at the same time.

Please refer to the FIG. 2 and FIG. 3. The water port 128 of the hydrogen water generator 12 of the present invention is located on the top of the container 120, that is, disposed on the cover 1202, and the hydrogen water generator 12 includes a water cover 1280 detachably covered on the water port 128. When the water cover 1280 is removed or opened from the water port 128, the hydrogen water generated in the containing space 1200 can be outputted to the outside of the container 120 through the water port 128. Alternatively, the supplemental water from outside of the container 120 can also enter to the containing space 1200 of the container 120 through the water port 128. In practice, the water cover 1280 can include a cover sheet for covering the water port 128 and a connecting strip connected to the cover sheet, so that the cover sheet can still be connected to the water cover 1280 through the connecting strip without falling or getting lost when the cover sheet is opened from the water port 128.

Moreover, the gas output port 126 of the hydrogen water generator 12 of the present invention can be disposed on the top of the container 120, that is, disposed on the cover 1202. As mentioned above, the gas comprising hydrogen forms the micro gas bubbles in the water after passing through the holes 1240 of the diffusing pipe 124 and the micro gas bubbles moves upward, and the gas bubbles are humidified in the process of moving upward. The humidified gas comprising hydrogen rises and gathers on the upper side of the containing space 1200, so that it can be collected by the gas output port 126 and outputted to the outside of the container 120. The gas output port 126 can directly provide the humidified gas comprising hydrogen (it can be called the humidified gas) to the patients for inhalation, and can be connected to a handheld or stationary nebulizer/vaporizer. The humidified gas comprising hydrogen and an atomized gas generated by the nebulizer/vaporizer (the atomized gas can be one of the groups consisting of water vapor, atomized potion, volatile essential oil and combinations thereof) form a mixed gas provided to the patients for inhalation.

In practice, the flow rate of the humidified gas comprising hydrogen outputted by the gas output port 126 can be between 0.01 L/min and 12 L/min. For example, the flow rate is between 1 L/min and 6 L/min for matching with the human respiration. In addition, in order to facilitate the user to take the hydrogen water, the hydrogen water generator 12 can also include a handle 121 (as shown in FIG. 1 and FIG. 2) disposed on the outer surface of the case 1202 of the container 120 for the user to hold to pour the hydrogen water.

The hydrogen production system may occur abnormal conditions during operation; for example, the hydrogen gas generating device 10 or the hydrogen water generator 12 is aged after long-term use to cause machine failure or leaks hydrogen due to improper operation by the user and other problems. These abnormal conditions may cause accidents, so that the hydrogen gas generating device 10 or the hydrogen water generator 12 needs a mechanism that can alert the user whether the current system is working normally or not. The present invention uses the container 120 emitting different color lights under the normal operation and the abnormal operation to remind the user. For example, the container 120 can emit blue light or green light when the hydrogen gas generating device 10 or the hydrogen water generator 12 is under the normal operating state, and the container 120 can emit yellow light or red light when the hydrogen gas generating device 10 or the hydrogen water generator 12 is under the abnormal operating state. Therefore, the user can directly determine whether the current system is working normally or not according to the color of the container 120. Moreover, the hydrogen gas generating device 10 or the hydrogen water generator 12 can also be specifically operated in response to system conditions to protect the entire system.

In detail, please refer to FIG. 2 again. As shown in FIG. 2, the position on the hydrogen gas generating device 10 for installing the hydrogen water generator 12 can be configured with a capacitive water level meter 100 and a luminous component 102. The capacitive water level meter 100 can detect the water level in the container 120 of the hydrogen water generator 12 when the hydrogen water generator 12 is assembled on the hydrogen gas generating device 10. In detail, the capacitive water level meter 100 includes two measuring sensors for measuring the high water level and the low water level in the container 120 respectively. Therefore, the user can know whether the water in the hydrogen water generator 12 should be replenished or not. On the other hand, when the hydrogen water generator 12 is assembled on the hydrogen gas generating device 10, the gas comprising hydrogen generated by the hydrogen gas generating device 10 passes the gas input port 122 into the containing space 1200 of the container 120. If the hydrogen water generator 12 is detached from the hydrogen gas generating device 10, the gas comprising hydrogen generated by the hydrogen gas generating device 10 may be leaked out to cause accident. The capacitive water level meter 100 of the hydrogen gas generating device 10 can avoid the aforementioned gas comprising hydrogen from leaking accident. In this embodiment, the hydrogen gas generating device 10 can include a controlling module connected to the capacitive water level meter 100. When the hydrogen water generator 12 is detached from the hydrogen gas generating device 10, the two measuring sensors of the capacitive water level meter 100 are unable to detect the high water level or the low water level in the container 120. Therefore, the capacitive water level meter 100 could not transmit the water level signal to the controlling module, or the controlling module actively checks if the capacitive water level meter 100 does not detect any water level, and then the controlling module controls the hydrogen gas generating device 10 to stop for generating the gas comprising hydrogen. Therefore, the controlling module and the capacitive water level meter 100 can prevent the hydrogen gas generating device 10 from leaking accident of the gas comprising hydrogen.

In the aforementioned embodiment, the water level meter is used to simultaneously measure the water level and detect whether the hydrogen water generator 12 is connected to the hydrogen gas generating device 10 to avoid leakage of gas comprising hydrogen. In practice, the position on the hydrogen gas generating device 10 for installing the hydrogen water generator 12 can be configured with an additional contact detecting unit for determining whether the hydrogen gas generating device 10 is connected to the hydrogen water generator 12 by detecting whether the container 120 is in contact with the contact detecting unit.

The luminous component 102 of the hydrogen gas generating device 10 can emit light toward the container 120 when the hydrogen water generator 12 is assembled on the hydrogen gas generating device 10. In this embodiment, the outer wall of the container 120 is transparent, so that the light emitted by the luminous component 102 can pass the container 120 into the containing space 1200. It should be noted that the position and number of the luminous component 102 are not limited in FIG. 2, the designs of the position and number can be determined by the user or requirements. Moreover, the luminous component 102 can be a light-emitting diode (LED) in practice, but is not limited thereto, any component that can emit light can be applied to the present invention.

The gas comprising hydrogen forms the micro gas bubbles in the water of the containing space 1200 and the micro gas bubbles moves upward, so that the light emitted from the luminous component 102 will be refracted or reflected by the micro gas bubbles after entering the containing space 1200, and then the light passes through the transparent outer wall to the external environment. Since the container 120 has the transparent outer wall, the user can observe the pipe wall of the diffusing pipe 124 generating the amount of micro gas bubbles suspending upward via the transparent outer wall, and the light emitted from the luminous component 102 emits to the micro gas bubbles to generate light refraction or reflection. Therefore, the user can feel the change of the light inside the container 120, thereby achieving the effect of visual beautification.

On the other hand, the luminous component 102 can also have different light emitting modes. In practice, the luminous component 102 can be controlled by the aforementioned controlling module to emit light with the light emitting modes. The luminous component 102 can emit lights with different colors or flash in different ways in the different light emitting modes. For example, when the flow rate of the gas comprising hydrogen entered the container 120 is greater, the luminous component 102 emits light with a higher flashing frequency. Furthermore, when the hydrogen gas generating device 10 and the hydrogen water generator 12 are operated under the normal states (such as the input rate of the gas comprising hydrogen in the normal range, the capacitive water level meter 100 under normal operation and so on), the luminous component 102 can emit a first color light, such as blue light or green light, to remind the user that the system is operated under normal state. However, when the hydrogen gas generating device 10 and the hydrogen water generator 12 are operated under the abnormal states (such as the input rate of the gas comprising hydrogen in the abnormal range or no gas comprising hydrogen inputted, or the capacitive water level meter 100 fault), the luminous component 102 can emit a second color light, such as yellow light or red light, to remind the user that the current system is operated under abnormal state or malfunctioning. Therefore, the luminous component 102 not only can generate different visual effects, but also can remind the user of the current system status, thereby ensuring the safety of the system and the user.

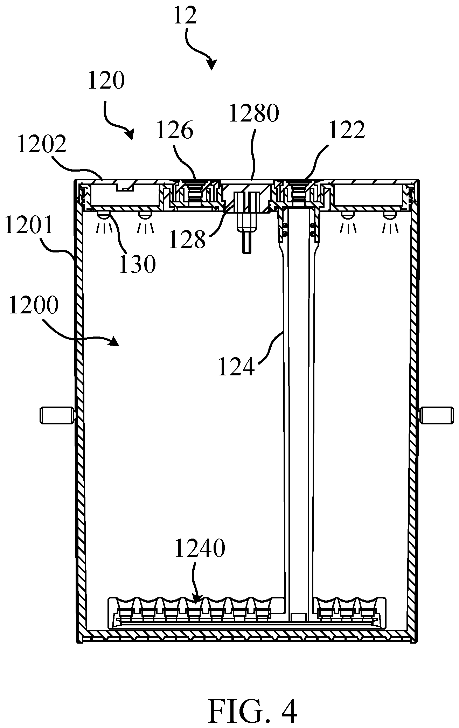

The aforementioned luminous component can also be disposed in the hydrogen water generator. Please refer to FIG. 4. FIG. 4 is an inside schematic diagram illustrating the hydrogen water generator 12 according to another embodiment of the present invention. As shown in FIG. 4, the hydrogen water generator 12 further includes the luminous component 130 disposed on the cover 1202 of the container 120 and can emit light toward the containing space 1200. The light can pass from the transparent outer wall of the container 120 to the external environment after being refracted or reflected by the micro gas bubbles. The luminous component 130 can be controlled by the controlling module of the hydrogen water generator 12 (not shown in figure), and can also be controlled by the device connected to the hydrogen water generator 12 (such as hydrogen gas generating device 10). The position and number of the luminous component 130 can be determined according to the design of the user or requirements. The luminous component 130 is the same as the luminous component 120 of aforementioned embodiment, which can generate different visual effects and remind the user that the current status of the system by different light emitting modes, thereby ensuring the safety of the system and the user. It should be noted that the structure and function of the other components of the hydrogen water generator 12 in this embodiment are the same as the structure and function of the corresponding component of the aforementioned embodiment, it will not be described herein.

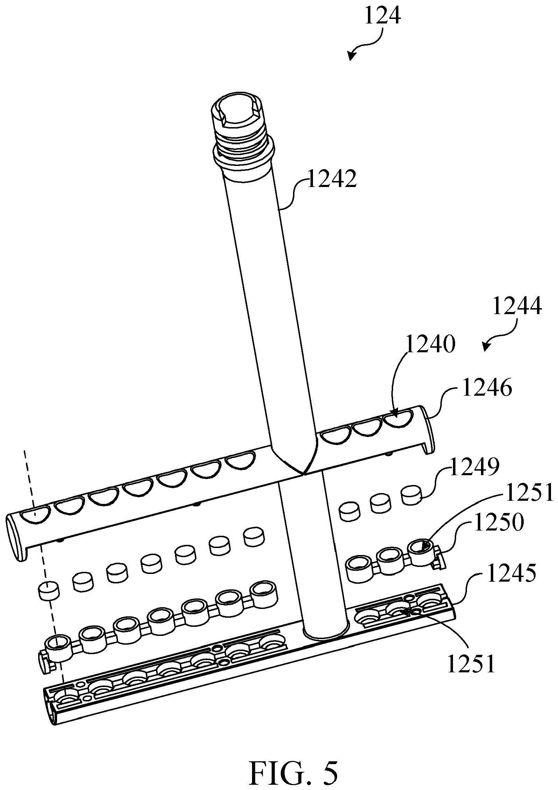

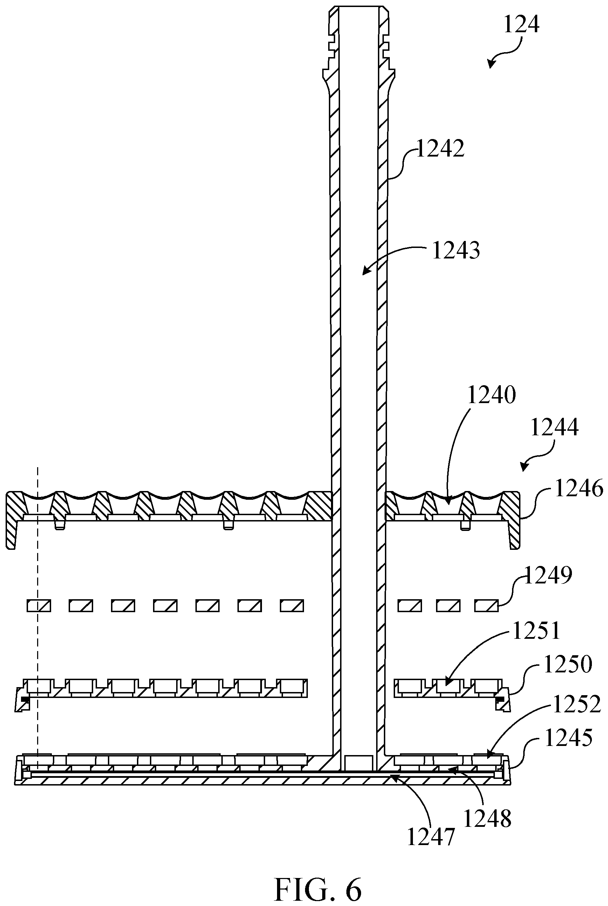

In the aforementioned embodiment, the diffusing pipe 124 can be configured to output the gas comprising hydrogen into the water of the containing space 1200 to form the micro gas bubbles. The following specification will describe the structure and function of the diffusing pipe 124 in detail. Please refer to FIG. 5 and FIG. 6. FIG. 5 is an exploded diagram illustrating the diffusing pipe 124 of FIG. 3. FIG. 6 is a sectional schematic diagram illustrating the diffusing pipe 124 of FIG. 5. As shown in FIG. 5 and FIG. 6, the diffusing pipe 124 includes a gas injecting tube 1242 and a gas injecting base 1244. One end of the gas injecting tube 1242 is coupled to the gas input port 122 and the gas injecting tube 1242 has a first gas injecting channel 1243. The gas injecting base 1244 can be disposed in the water of the containing space 1200 and further include a gas injecting base body 1245 and a gas injecting cover 1246. The gas injecting base body 1245 is coupled to the gas injecting tube 1242 and has a second gas injecting channel 1247 and a plurality of gas injecting holes 1248. The second gas injecting channel 1247 is coupled to the first gas injecting channel 1243, and the gas injecting holes 1248 are coupled to the second gas injecting channel 1247. The gas injecting cover 1246 is embedded to the gas injecting base body 1245. The gas injecting cover 1246 includes the plurality of holes 1240 corresponding to the gas injecting holes 1248 respectively, so that the holes 1240 are coupled to the second gas injecting channel 1247 through the gas injecting holes 1248. When the gas comprising hydrogen enters the diffusing pipe 124 from the gas input port 122, the gas comprising hydrogen flows through the first gas injecting channel 1243, the second gas injecting channel 1247 and the gas injecting holes 1248 in sequence, and then enters into the water in the containing space 1200 through the holes 1240 to form the bubble state.

In practice, the size of the holes 1240 can be determined as the user or requirements, and the bubble size formed by the gas comprising hydrogen can be changed according to the holes 1240. Moreover, in this embodiment, the diffusing pipe 124 also can include a plurality of micro filters 1249 disposed between the holes 1240 and the gas injecting holes 1248 respectively for filtering the gas comprising hydrogen which passing through the holes 1240, to ensure the safety and quality of the gas comprising hydrogen injected into the water. The micro filter 1249 can further divide the gas comprising hydrogen into micro gas bubbles to increase the contact area of the gas comprising hydrogen and water, thereby increasing the concentration of the gas comprising hydrogen dissolved in water. Therefore, the bubble size formed by the gas comprising hydrogen of this embodiment can be controlled by the micro filter 1249 instead of the holes 1240. In practice, the micro filter 1249 can be an activated carbon filter, a drinking water filter and so on, but it is not limited thereto. Furthermore, the diffusing pipe 124 further includes a fixing component 1250 having a plurality of fixing holes 1251 for containing and fixing the micro filters 1249, and the surface of the gas injecting base body 1245 facing to the gas injecting cover 1246 has a groove 1252 for containing the fixing component 1250.

As shown in FIG. 5, the hole 1240 is a hollow dome structure, and the area of the opening of the hole 1240 toward the containing space 1200 is greater than the area of the opening of the hole 1240 toward the gas injecting holes 1248. Therefore, the hole 1240 can increase dispersion of micro gas bubbles of the gas comprising hydrogen injected into water. On the contrary, if the area of the opening of the hole 1240 toward the containing space 1200 is smaller than the area of the opening of the hole 1240 toward the gas injecting holes 1248, the micro gas bubbles of the gas comprising hydrogen will be gathered into a large gas bubbles, thereby reducing the contact area of the gas comprising hydrogen in the drinking water.

In order to evenly distribute the amount of the gas comprising hydrogen flowing out of each hole 1240, to improve the output efficiency of the micro gas bubble of the gas comprising hydrogen and make the water in the containing space 1200 have a uniform dissolved hydrogen, the cross-sectional area of the second gas injecting channel 1247 gradually increases from the coupling position with the first gas injecting channel 1243 to the two ends of the gas injecting base body 1245. Therefore, the shape of the second gas injecting channel 1247 is designed with narrow middle and wide ends, which can increase the flow rate of the gas comprising hydrogen from the coupling position to the two ends, so as to prevent most of the gas comprising hydrogen from being injected into the water near the coupling position with the first gas injecting channel 1243 and all the holes 1240 cannot be fully utilized.

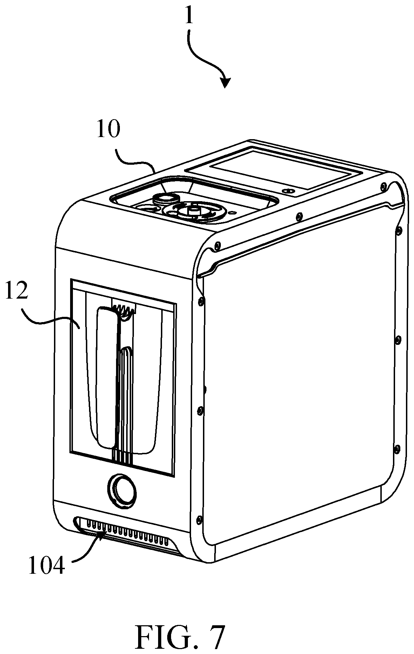

As described in the previous embodiments, the luminous component 102 disposed on the hydrogen gas generating device 10 or the luminous component 130 disposed on the hydrogen water generator 12 can have various light emitting modes to emit different color lights to remind the user whether the current system is operated under normal state, such as the leakage of the gas comprising hydrogen. The hydrogen gas generating system of the present invention also can include a device for detecting the hydrogen concentration of the outside of the hydrogen gas generating device to deal with the above abnormal conditions. Please refer to FIG. 7. FIG. 7 is a schematic diagram illustrating the hydrogen gas generating system 1 according to another embodiment of the present invention. As shown in FIG. 7, the difference between this embodiment and the aforementioned embodiment is that the hydrogen gas generating device 10 of the hydrogen gas generating system 1 in this embodiment further includes a hydrogen concentration detecting device 104 for detecting the hydrogen concentration of the outside of the hydrogen gas generating device 10. It should be noted that the structure and function of the other components of the hydrogen gas generating system 1 in this embodiment are the same as the structure and function of the corresponding component of the aforementioned embodiment, it will not be described herein. Therefore, in the present invention, the hydrogen gas generating system can include the hydrogen gas generating device, the hydrogen water generator (including the container, the handle, the gas input port, the diffusing pipe, the gas output port, the water port and so on), the hydrogen gas detecting device and a warning device to warn the user whether the hydrogen gas is leaked out by the system or not. In one embodiment, the warning device can be the aforementioned luminous component 102 disposed on the hydrogen gas generating device 10, which generates a warning signal (the second color light) by emitting light to the container 120 of the hydrogen water generator 12 with the transparent outer wall. In another embodiment, the warning device also can be the aforementioned luminous component 130 disposed on the container 120, which generates a warning signal (the second color light) by emitting light to the container 120 of the hydrogen water generator 12 with the transparent outer wall. In addition, the warning device also can be a sound generating component disposed on the hydrogen gas generating device or the hydrogen water generator. The sound generating component can generate a warning sound as the warning signal under the aforementioned abnormal conditions, to remind the user that the system is under the abnormal state.

In the embodiment of FIG. 7, the controlling module of the hydrogen gas generating device 10 can compare the external hydrogen concentration detected by the hydrogen concentration detecting device 104 with a preset hydrogen concentration. When the hydrogen concentration of the outside of the hydrogen gas generating system 1 is higher than the preset hydrogen concentration, the controlling module determines that the hydrogen gas is leaking, and then the controlling module controls the luminous component 102 disposed on the hydrogen gas generating device 10 or the luminous component 130 disposed on the hydrogen water generator 12 to emit the aforementioned second color light, such as yellow light or red light, to remind the user that the current system is operated under the abnormal state or malfunction. Relatively, if the hydrogen concentration of the outside of the hydrogen gas generating system 1 is lower than the preset hydrogen concentration, the controlling module can control the luminous component 102 disposed on the hydrogen gas generating device 10 or the luminous component 130 disposed on the hydrogen water generator 12 to emit the aforementioned first color light, such as blue light or green light, to remind the user that the current system is operated under the normal state. It should be noted that the hydrogen concentration of the outside of the hydrogen gas generating system 1 lower than the preset hydrogen concentration does not mean the system operated under the normal state in practice; the hydrogen gas generating system 1 may still have abnormal states or malfunctions other than hydrogen leakage. If the controlling module detects those abnormal states or malfunctions, the controlling module will control the luminous component 102 or the luminous component 130 to emit the second color light to remind the user that the current system is operated under the abnormal state or malfunction.

When the controlling module determines that the hydrogen gas generating system 1 has the hydrogen gas leakage condition according to the external hydrogen concentration detected by the hydrogen concentration detecting device 104, the controlling module not only can change the light emitting mode of the luminous component 102 or the luminous component 130, but also can stop the hydrogen gas generating device 10 for generating the hydrogen gas to prevent the accident.

The above-mentioned hydrogen concentration detecting device not only can be disposed on the hydrogen gas generating device, but also can be disposed on the hydrogen water generator 12, such as disposed on the container 120, to directly control the luminous component 102 or the luminous component 130 to emit the second color light. The hydrogen concentration detecting device disposed on the hydrogen water generator 12 also can be electrically connected to the controlling module of the hydrogen gas generating device 10 when the hydrogen water generator 12 is assembled on the hydrogen gas generating device 10 and transmit the detecting result to the controlling module. Then, the controlling module controls the light emitting mode of the luminous component or stops the hydrogen gas generating device 10 for generating the hydrogen according to the detecting result.

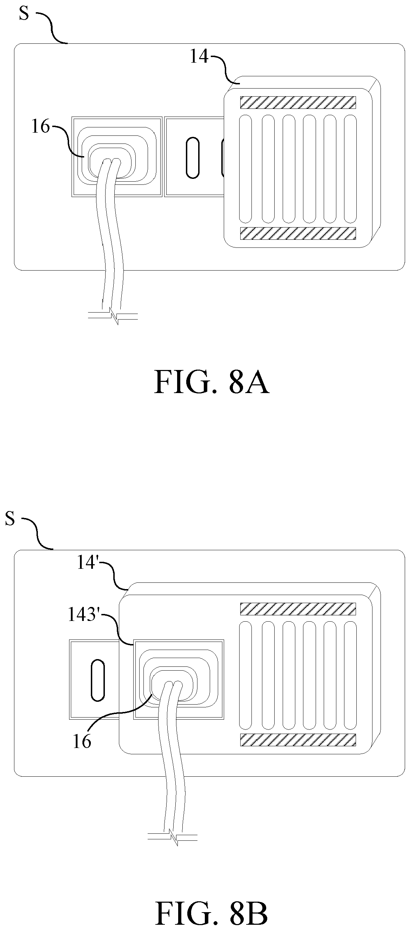

In addition, the hydrogen concentration detecting device further can be disposed outside of the hydrogen gas generating device and the hydrogen water generator. Please refer to FIG. 8A and FIG. 8B. FIG. 8A is a schematic diagram illustrating an off-board hydrogen detecting component 14 according to an embodiment of the present invention. FIG. 8B is a schematic diagram illustrating the off-board hydrogen detecting component 14' according to another embodiment of the present invention. As shown in FIG. 8A, the off-board hydrogen detecting component 14 can be a structure with a plug (not shown in figure), which directly inserted into the power socket S adjacent to the power plug 16 of the hydrogen gas generating device 10. As shown in FIG. 8B, the off-board hydrogen detecting component 14' can be a structure with a plug and a socket 143' which can be directly inserted into the power socket S and provided for the power plug 16 of the hydrogen gas generating device 10 inserting into the socket 143' of the off-board hydrogen detecting component 14'. In the above-mentioned embodiment, the off-board hydrogen detecting component 14 can be one part of the hydrogen gas generating system 1. In the embodiment of FIG. 8A, the off-board hydrogen detecting component 14 can be communicated with the hydrogen gas generating device 10 to transmit the external hydrogen concentration to the controlling module of the hydrogen gas generating device 10 for comparison, so as to determine whether the hydrogen concentration of the outside of the hydrogen gas generating system 1 exceeds the standard or not. Alternatively, the off-board hydrogen detecting component 14 can directly determine whether the hydrogen concentration of the outside of the hydrogen gas generating system 1 exceeds the standard or not to inform the controlling module of the hydrogen gas generating device 10 to control the luminous component to change the light emitting mode and stop for generating the hydrogen gas. In the embodiment of FIG. 8B, in addition to the aforementioned functions, the off-board hydrogen detecting component 14' further can directly cut off the power of the power socket S when the hydrogen concentration of the outside of the hydrogen gas generating system 1 exceeds the standard. Therefore, the hydrogen gas generating device 10 can be directly shut down without going through the control unit of the hydrogen production device 10.

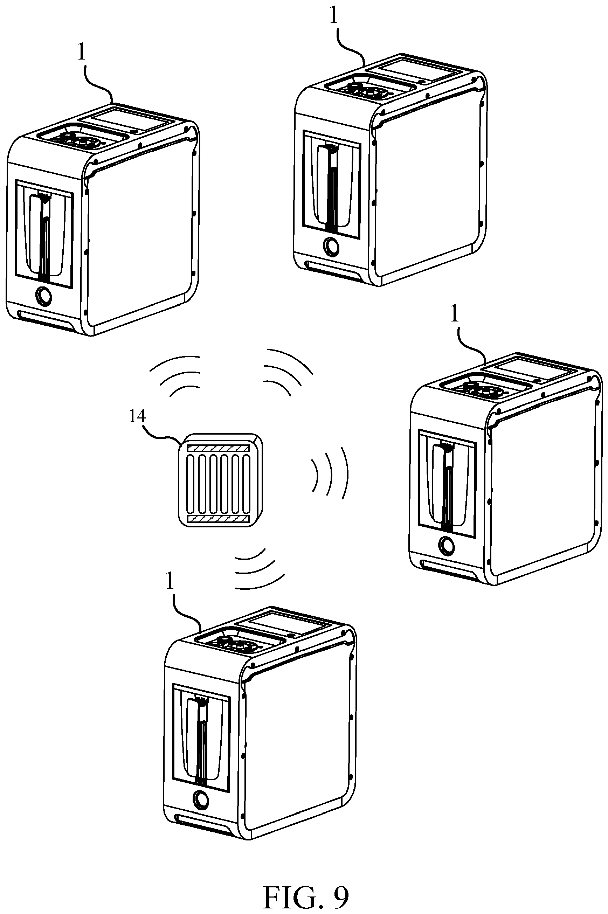

Please refer to FIG. 9. FIG. 9 is a schematic diagram illustrating the cooperation of the off-board hydrogen detecting component 14 and a plurality of hydrogen gas generating systems 1 according to another embodiment of the present invention. As shown in FIG. 9, the off-board hydrogen detecting component 14 also can be communicated with the controlling modules of the hydrogen production devices 10 of the hydrogen gas generating systems 1 simultaneously. It should be noted that the FIG. 9 shows the cooperation of the off-board hydrogen detecting component 14 and four hydrogen gas generating systems 1, but the number of the hydrogen gas generating systems 1 is not limited to four in practice. In this embodiment, these hydrogen gas generating systems 1 and the off-board hydrogen detecting component 14 are located in the same area, so that the off-board hydrogen detecting component 14 can detect the hydrogen concentration of outside of the hydrogen gas generating systems 1. When the off-board hydrogen detecting component 14 detects that the hydrogen concentration exceeds the standard, the luminous component of each hydrogen gas generating systems 1 will change the light emitting mode and stop for generating hydrogen gas, and even each of the hydrogen gas generating systems 1 will be shut down directly, to ensure the safety of the hydrogen gas generating systems 1 and the user.

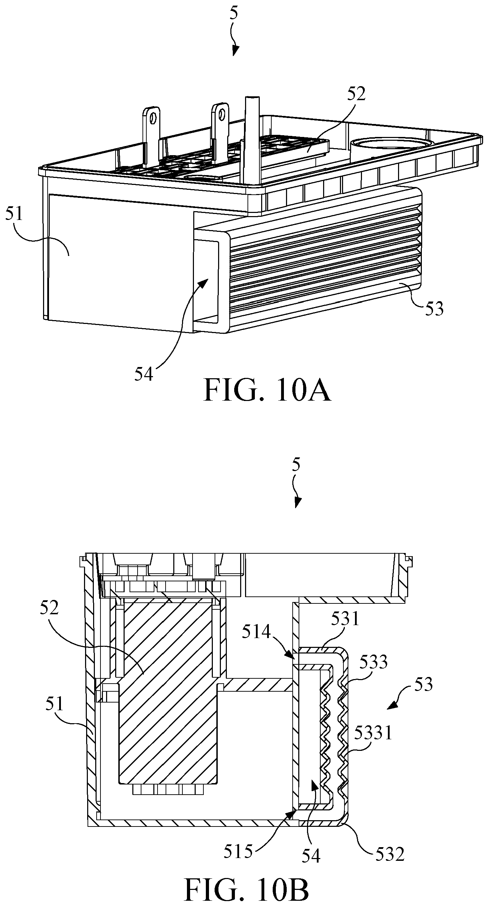

The following specification will describe the internal structure of hydrogen gas generating device of the above-mentioned embodiment. Please refer to FIG. 10A and FIG. 10B. FIG. 10A is an assembly diagram illustrating a water tank 51, an electrolysis module 52 and a heat dissipater 53 of the hydrogen gas generating device 5 according to an embodiment of the present invention. FIG. 10B is a sectional diagram illustrating the water tank 51, the electrolysis module 52 and the heat dissipater 53 of FIG. 10A. As shown in FIG. 10A and FIG. 10B, in this embodiment, the electrolysis module 52 is disposed in the water tank 51 and configured to electrolyze the water from the water tank 51 to generate the gas comprising hydrogen. The water inlet tube 531, the water outlet tube 532 and the tubular structure 533 of the heat dissipater 53 are all flat tube structures. The shapes of the first opening 514 and the second opening 515 of the water tank 51 are corresponding to the shapes of the water inlet tube 531 and the water outlet tube 532 respectively, so that the internal space of the water tank 51 is communicated with the inside of the water inlet tube 531, the water outlet tube 532 and the tubular structure 533 of the heat dissipater 53. In practice, as shown in FIG. 10A, the widths of the water inlet tube 531, the water outlet tube 532 and the tubular structure 533 can be equal to the length of the side surface of the water tank 51. That is to say, the first opening 514 and the second opening 515 of the water tank 51 have a larger area and coverage. Therefore, when the hydrogen gas generating device is operating, the electrolyzed water comprising thermal energy can flow to the heat dissipater 53 in a large amount and quickly through the first opening 514 of the water tank 51. Then, the electrolyzed water comprising thermal energy flows through the tubular structure 533 for dissipating the heat, and the electrolyzed water after dissipating also can rapidly flow to the water tank 51 through the second opening 515, thereby increasing the heat dissipating efficiency. The widths of the water inlet tube 531, the water outlet tube 532 and the tubular structure 533 of the heat dissipater 53 are not limited thereto, the widths of those components can also be any length or determined as requirement. Furthermore, in this embodiment, the tubular structure 533 includes a delay structure 5331 with wave shape for extending the time of the electrolyzed water staying in the tubular structure 533, thereby increasing the heat dissipating efficiency. In practice, the shape and configuration of the delay structure 5331 are not limited thereto.

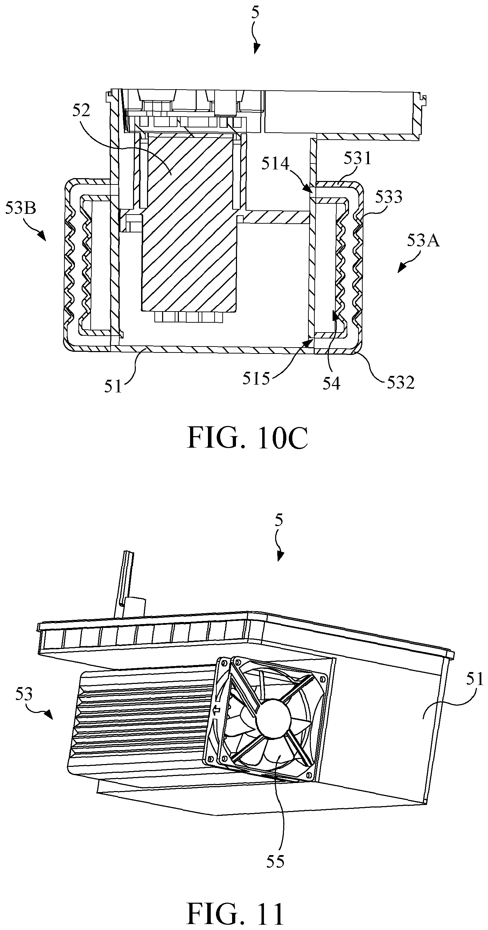

The heat dissipater of the hydrogen gas generating device of the present invention not only can be disposed on the right side of the water tank (as shown in FIG. 10A), it can be disposed on the left side of the water tank. Moreover, the number and configuration of the heat dissipater of the present invention are not limited thereto. Please refer to FIG. 10C. FIG. 10C is an assembly diagram illustrating the water tank 51, the electrolysis module 52 and the heat dissipater 53 of the hydrogen gas generating device 5 according to another embodiment of the present invention. In this embodiment, the hydrogen gas generating device 5 also can include two heat dissipaters 53 disposed on left side and right side of the water tank 51 respectively. The functions of the water tank 51, the electrolysis module 52 and the heat dissipater 53 in this embodiment are the same as functions of the corresponding components of the aforementioned embodiment, it will not be described herein. Moreover, the hydrogen gas generating device can further include a plurality of fins disposed on the outside of the tubular structure to increase the heat dissipating efficiency.

Please refer to FIG. 10A, FIG. 10B and FIG. 11. FIG. 11 is a structural schematic diagram illustrating the water tank 51, the heat dissipater 53 and the fan 55 of the hydrogen gas generating device 5 in another one perspective. As shown in FIG. 10A, FIG. 10B and FIG. 11, when the heat dissipater 53 is connected to the water tank 51, the water inlet tube 531, the water outlet tube 532 and the tubular structure 533 of the heat dissipater 53 and the sidewall of the water tank 51 form a heat dissipating duct 54. The hydrogen gas generating device 5 can further include a fan 55 corresponding to the heat dissipating duct 54 to guide gas from the external environment into the heat dissipating duct 54. In practice, when the heat dissipater 53 is operating, the thermal energy generated by the electrolyzed water will be transmitted to the tubular structure 533 of the heat dissipater 53. At this time, the fan 55 can guide the external gas into the heat dissipating duct 54 to reduce the thermal energy and temperature of the tubular structure 533. It should be noted that the number and configuration of the fan of the hydrogen gas generating device are not limited thereto. In one embodiment, the fan can include a first fan and a second fan disposed on two ends of the heat dissipating duct respectively. The first fan can guide gas from the external environment into the heat dissipating duct, and the second fan can guide gas in the heat dissipating duct to the external environment. In another embodiment, when the hydrogen gas generating device includes two heat dissipaters disposed on the left side and right side of the water tank respectively, the hydrogen gas generating device also can include two set of fans disposed on the left side and right side of the water tank respectively and corresponding to the heat dissipating ducts formed by the two heat dissipaters.

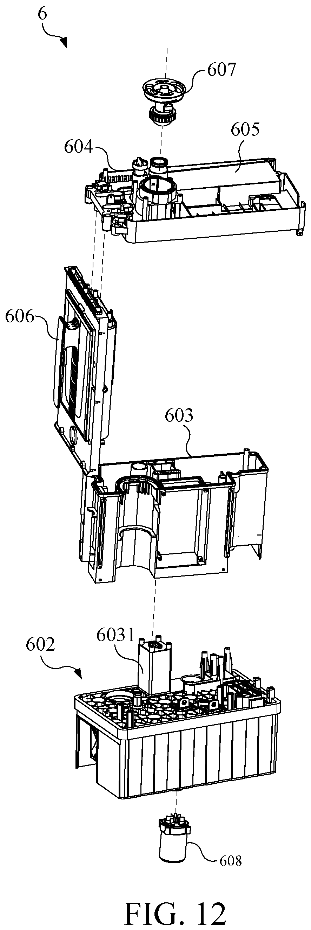

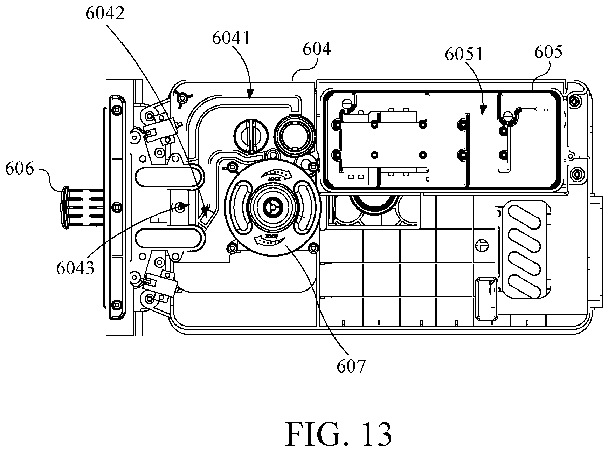

Please refer to FIG. 12 and FIG. 13. FIG. 12 is a simple exploded diagram illustrating the hydrogen gas generating device 6 according to an embodiment of the present invention. FIG. 13 is a simple schematic diagram illustrating the hydrogen gas generating device 6 in another one perspective of FIG. 12. As shown in FIG. 12 and FIG. 13, the hydrogen gas generating device 6 of this embodiment includes an electrolysis module (not shown in figure), a water tank 602, a humidifying module 603, an integrated passageway module 604, a condensing and filtering module 605, a hydrogen water cup 606 and a nebulizer 607. The humidifying module 603 is vertically disposed above the water tank 602, the integrated passageway module 604 is vertically disposed above the humidifying module 603, and the condensing and filtering module 605 is disposed in the containing space of the integrated passageway module 604.

The condensing and filtering module 605 can be configured to filter the gas comprising hydrogen and include a condensing flow channel 6051. In practice, the condensing and filtering module 605 can be embedded in the integrated passageway module 604, and can be pulled out from the integrated passageway module 604 to facilitate replacement without disassembling the entire hydrogen gas generating device 6 for replacement, but it is not limited thereto. The condensing and filtering module 605 also can be disposed in the integrated passageway module 604 in a fixed manner. The humidifying module 603 includes a humidifying chamber (not shown in figure) and a connecting chamber 6031. The humidifying chamber contains the supplemental water for humidifying the gas comprising hydrogen. The connecting chamber 6031 can be configured for connecting the water tank 602 and the integrated passageway module 604, so that the gas comprising hydrogen generated by the electrolysis module that disposed in the water tank 602 can flow into the condensing flow channel 6051 of the condensing and filtering module 605. The hydrogen water cup 606 is configured to contain the drinking water, and the hydrogen water cup 606 is configured for injecting the gas comprising hydrogen to the drinking water to form the hydrogen water. The integrated passageway module 604 includes a gas inlet channel 6041, a gas outlet channel 6042 and a gas connecting channel 6043. Wherein, the gas inlet channel 6041 and the gas outlet channel 6042 can be selectively coupled to the hydrogen water cup 606, and the gas connecting channel 6043 can be selectively coupled to the gas inlet channel 6041 and the gas outlet channel 6042. The nebulizer 607 can be coupled to the gas outlet channel 6042 of the integrated passageway module 604 to receive the gas comprising hydrogen, and can generate an atomized gas and mix the atomized gas with the gas comprising hydrogen to form a health gas. Moreover, the humidifying module 603, the condensing and filtering module 605, the hydrogen water cup 606 and the nebulizer 607 can also be embedded or directly coupled to the integrated passageway module 604.

Therefore, when the gas comprising hydrogen generated by the electrolysis module leaves the water surface of the water tank 602, it will enter to the connecting chamber 6031 of the humidifying module 603 rapidly. Then, the gas comprising hydrogen sequentially flows through the connecting chamber 6031 of the humidifying module 603, the condensing flow channel 6051 of the condensing and filtering module 605, the gas inlet channel 6041 and the gas outlet channel 6042 of the integrated passageway module 604 and the nebulizer 607. Wherein, the gas comprising hydrogen selectively flows through the hydrogen water cup 606. However, it should be noted that the flow direction of the above-mentioned gas comprising hydrogen is one of the embodiments of the hydrogen gas generating device of the present invention. Those skilled in the art can adjust the order of each component according to requirements, which is not limited thereto.

The nebulizer 607 can generate an atomized gas and mix the atomized gas with the gas comprising hydrogen to form a health gas. Wherein, the atomized gas can be one of the groups consisting of water vapor, atomized potion, volatile essential oil and combinations thereof. In one embodiment, the nebulizer 607 includes an oscillator. The oscillator atomizes the water, atomized potion or volatile essential oil added to the nebulizer 607 by shaking to generate the atomized gas, and then the nebulizer 607 mixes the mixed gas with the atomized gas to form the health gas. The nebulizer 607 can selectively be turned on or off according to the user's requirements to provide the health gas mixed with the atomized gas to the user for inhalation, or only provide the mixed gas (the gas comprising hydrogen diluted by the second oxygen) to the user for inhalation.

As shown in FIG. 12, in one embodiment, the hydrogen gas generating device 6 includes a water pump 608 disposed on the bottom of the water tank 602 and connected to the lower cavity of the water tank 602. In practice, the water pump 608 can guide the electrolyzed water located in the lower cavity of the water tank 608 into the electrolysis module through the fan portion, so as to improve the electrolysis efficiency. Moreover, since the water pump 608 accelerates the flow of the electrolyzed water from the lower cavity to the upper cavity, and the electrolyzed water in the upper cavity is also accelerated to flow into the heat dissipater through the first opening for heat dissipation, thereby improving the heat dissipating efficiency.

Therefore, the hydrogen gas generating device of the present invention can isolate the electrolyzed water comprising thermal energy to the upper cavity of the water tank through the separator plate and can effectively dissipate heat by the heat dissipater connecting the upper cavity and the lower cavity. Moreover, the hydrogen gas generating device of the present invention can reduce the thermal energy of the heat dissipater through the fan disposed on the heat dissipating duct, thereby increasing the heat dissipating efficiency. Furthermore, the hydrogen gas generating device of the present invention also can initiatively guide the electrolyzed water of the water tank into the heat dissipater by the water pump for heat dissipation to increase the heat dissipating efficiency. In addition, the hydrogen gas generating device can improve the heat dissipating efficiency by the structure with extending the path length in the tubular structure of the heat dissipater.

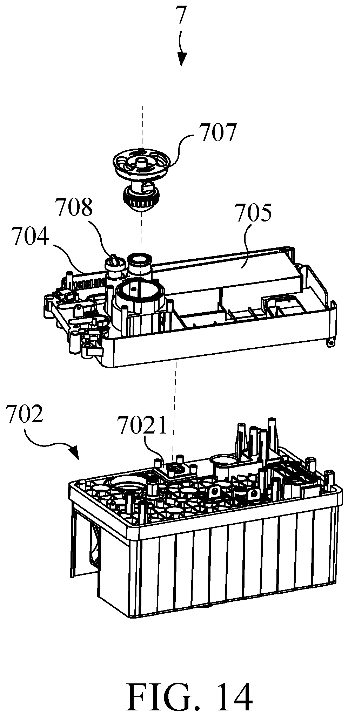

Moreover, the configuration of the hydrogen gas generating device of the present invention not only can be the above-mentioned type, it also can be other type. Please refer to FIG. 14. FIG. 14 is a simple exploded diagram illustrating the hydrogen gas generating device 7 according to an embodiment of the present invention. As shown in FIG. 14, the difference between this embodiment and aforementioned embodiment is that the hydrogen gas generating device 7 of this embodiment only includes the water tank 702, the integrated passageway module 704, the condensing and filtering module 705 and the nebulizer 707. The integrated passageway module 704 is vertically disposed above the water tank 702, the condensing and filtering module 705 is disposed in the integrated passageway module 704, and the nebulizer 707 is connected to the integrated passageway module 704. When the gas comprising hydrogen generated by the electrolysis module of the water tank 702 leaves the water surface of the water tank 702, it will enter to the connecting chamber 7021 rapidly. Then, the gas comprising hydrogen sequentially flows through the connecting chamber 7021, the condensing flow channel of the condensing and filtering module 705, the gas inlet channel and the gas outlet channel of the integrated passageway module 704 and the nebulizer 707.

Furthermore, the hydrogen gas generating device 7 of the present invention further includes a water supplemental port 708 disposed on the integrated passageway module 704 and connected to the flow channel of the integrated passageway module 704. In practice, the water supplemental port 708 can be connected to the gas outlet channel of the integrated passageway module 704. Since the water of the water tank 702 will gradually decrease after the hydrogen gas generating device 7 reacts for a long time, the user can add water through the water supplemental port 708 to supplement the water in the water tank 702. When the user replenishes the water in the water tank 702, the supplement water will flow through the condensing flow channel of the condensing and filtering module 705 and the connecting chamber 7021 to the water tank 702. Moreover, when the condensing and filtering module 705 is activated, the condensing and filtering module 705 will filter the electrolyte in the electrolyzed water, so that the electrolyte remains in the condensing flow channel. Therefore, when the user replenishes water from the water supplemental port 708 and the supplemental water flows through the condensing flow channel, the electrolyte located in the condensing flow channel can also be flushed back into the water tank 702 with the supplemental water. It should be noted that the configuration of the water supplemental port 708 is not limited thereto. In one embodiment, the water supplemental port can be connected to the nebulizer. In another embodiment, the water supplemental port can be directly connected to the condensing flow channel of the condensing and filtering module.

In summary, the hydrogen water generator and the hydrogen gas generating system can simply generate the hydrogen water and the humidified gas comprising hydrogen (or humidified gas), and the hydrogen water does not contain excess minerals or magnesium oxide, so that the hydrogen water does not need the filtering process and can be maintained high solubility. Moreover, the luminous component of the hydrogen water generator that irradiates light to the micro gas bubbles in the container further can produce visual effects on the appearance, so that it will stimulate consumers' desire to buy, and the user can understand the current operating status of the system. Furthermore, the capacitive water level meter of the hydrogen gas generating device not only can measure the water level in the hydrogen water generator, but also can detect whether the hydrogen water generator is detached from the hydrogen gas generating device to stop generating the gas comprising hydrogen, which ensures the safety of the system and the user. The hydrogen gas generating system further includes the on-board or the off-board hydrogen concentration detecting device for detecting whether the gas comprising hydrogen is being leaked or the hydrogen concentration of the environment exceeds the standard, thereby reminding the user or directly shutting down the system to avoid accidents.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A hydrogen water generator with warning function, comprising:
a container, comprising a case having an opening and a cover configured on the opening, the case forming a containing space to contain water;
a handle, configured on the case of the container for a user to hold;
a gas input port, configured on the container to receive a gas comprising hydrogen from the outside of the container;
a diffusing pipe, configured in the containing space and coupled to the gas input port to receive the gas comprising hydrogen, a part of the diffusing pipe being configured in the water of the containing space, and a pipe wall of the diffusing pipe having a plurality of holes, the gas comprising hydrogen passing from the diffusing pipe into the water of the containing space through the holes to generate a hydrogen water and a humidified gas comprising hydrogen;
a gas output port, configured on the container to receive the humidified gas comprising hydrogen from the containing space and output the humidified gas comprising hydrogen to the outside of the container;
a water port, configured on the container to output the hydrogen water of the containing space to the outside of the container; and
a luminous component, configured to emit light toward the hydrogen water generator from the outside of the hydrogen water generator, and the emitted light passing through the container to emit light from the container, the luminous component being a LED for showing the first color light under the normal state of the hydrogen water generator and showing the second color light under the abnormal state of the hydrogen water generator.

2. The hydrogen water generator of claim 1, wherein the container has a transparent outer wall, the luminous component is configured on the container to emit light toward the inside of the hydrogen water generator, and the emitted light is emitted from the container through the transparent outer wall.

3. The hydrogen water generator of claim 1, wherein the gas input port, the gas output port and the water port are configured on the cover.

4. The hydrogen water generator of claim 1, further comprising a water cover detachably covering on the water port, the water cover comprising a cover sheet and a connecting strip.

5. The hydrogen water generator of claim 1, wherein the diffusing pipe further comprises a plurality of micro filters corresponding to the holes respectively, the micro filters are configured to filter the gas comprising hydrogen passing through the holes, and the gas comprising hydrogen forms a plurality of micro gas bubbles after entering into the water of the containing space through the holes.

6. The hydrogen water generator of claim 1, further comprising a hydrogen concentration detecting device configured to detect whether a hydrogen concentration of the outside of the container is higher than a preset hydrogen concentration, the container showing the first color light when the hydrogen concentration is lower than the preset hydrogen concentration, and the container showing the second color light when the hydrogen concentration is higher than the preset hydrogen concentration.

7. A hydrogen gas generating system, comprising:
a hydrogen gas generating device, configured to generate a gas comprising hydrogen;
a hydrogen water generator, further comprising:
a container, comprising a case having an opening and a cover configured on the opening, the case forming a containing space to contain water, and the container being detachably configured on the hydrogen gas generating device;
a handle, configured on the case of the container for a user to hold;
a gas input port, configured on the container to receive the gas comprising hydrogen from the hydrogen gas generating device;
a diffusing pipe, configured in the containing space and coupled to the gas input port to receive the gas comprising hydrogen, a part of the diffusing pipe being configured in the water of the containing space, and a pipe wall of the diffusing pipe having a plurality of holes, the gas comprising hydrogen passing from the diffusing pipe into the water of the containing space through the holes to generate a hydrogen water and a humidified gas comprising hydrogen;
a gas output port, configured on the container to receive the humidified gas comprising hydrogen from the containing space and output the humidified gas comprising hydrogen to the outside of the container; and
a water port, configured on the container to output the hydrogen water to the outside of the container;
a hydrogen gas detecting device, configured to detect whether a hydrogen concentration is higher than a preset hydrogen concentration; and
a warning device, coupled to the hydrogen gas detecting device and configured to generate a warning signal when the hydrogen concentration detected by the hydrogen gas detecting device is higher than the preset hydrogen concentration.

8. The hydrogen gas generating system of claim 7, wherein the warning device is a LED luminous component configured on the hydrogen gas generating device, the LED luminous component causes the container to show a first color light when the hydrogen concentration detected by the hydrogen gas detecting device is lower than the preset hydrogen concentration, and the LED luminous component generates the warning signal to cause the container to show a second color light when the hydrogen concentration detected by the hydrogen gas detecting device is higher than the preset hydrogen concentration.

9. The hydrogen gas generating system of claim 7, wherein the warning device is a LED luminous component configured on the hydrogen water generator, the LED luminous component causes the container to show a first color light when the hydrogen concentration detected by the hydrogen gas detecting device is lower than the preset hydrogen concentration, and the LED luminous component generates the warning signal to cause the container to show a second color light when the hydrogen concentration detected by the hydrogen gas detecting device is higher than the preset hydrogen concentration.

10. The hydrogen gas generating system of claim 7, wherein the hydrogen gas detecting device is configured in the hydrogen gas generating device to detect whether the hydrogen concentration in the hydrogen gas generating device is higher than the preset hydrogen concentration, the warning device generates the warning signal to cause the container to show a second color light when the hydrogen concentration detected by the hydrogen gas detecting device is higher than the preset hydrogen concentration.

11. The hydrogen gas generating system of claim 7, wherein the hydrogen gas detecting device is configured outside of the hydrogen gas generating device, the hydrogen gas detecting device is coupled to the hydrogen gas generating device by wireless transmission to detect whether the hydrogen concentration around the hydrogen gas generating device is higher than the preset hydrogen concentration, the warning device generates the warning signal to cause the container to show a second color light when the hydrogen concentration detected by the hydrogen gas detecting device is higher than the preset hydrogen concentration.

12. The hydrogen gas generating system of claim 7, further comprising a plurality of associated hydrogen gas generating devices, each of associated hydrogen gas generating devices comprising an associated container and an associated warning device, wherein the associated warning device is configured on a case or the associated container of the associated hydrogen gas generating device, the hydrogen gas detecting device is coupled to the associated hydrogen gas generating devices by wireless transmission, when the hydrogen concentration around the hydrogen gas generating device or the hydrogen concentration around the associated hydrogen gas generating devices detected by the hydrogen gas detecting device is higher than the preset hydrogen concentration, the warning device and the associated warning devices cause the container and the associated containers to show a second color light respectively.

13. The hydrogen gas generating system of claim 7, wherein the warning device is a sound generating component configured to generate a warning sound as the warning signal when the hydrogen concentration detected by the hydrogen gas detecting device is higher than the preset hydrogen concentration.

14. The hydrogen gas generating system of claim 7, wherein the hydrogen gas generating device further comprises a capacitive water level meter configured to detect the water level of the water contained in the container of the hydrogen water generator when the container is configured on the hydrogen gas generating device.

15. The hydrogen gas generating system of claim 14, wherein the hydrogen gas generating device further comprises a controlling module connected to the capacitive water level meter, the capacitive water level meter comprises a high water level sensor and a low water level sensor, when the container is detached from the hydrogen gas generating device to cause the high water level sensor and the low water level sensor unable to detect a high water level and a low water level of the container, the controlling module controls the hydrogen gas generating device to stop for generating the gas comprising hydrogen.

16. The hydrogen gas generating system of claim 7, wherein the hydrogen gas generating device further comprises:
a water tank, configured for containing a water;
an electrolysis module, configured in the water tank and configured to electrolyze the water to generate the gas comprising hydrogen;
an integrated passageway module, vertically configured above the water tank and connected to the water tank to receive the gas comprising hydrogen;
a condensing and filtering module, coupled to the integrated passageway module to receive and filter the gas comprising hydrogen from the integrated passageway module; and
a humidifying module, vertically configured above the water tank and located between the water tank and the integrated passageway module, the humidifying module being coupled to the integrated passageway module to receive and humidify the filtered gas comprising hydrogen.

17. The hydrogen gas generating system of claim 16, wherein the condensing and filtering module has a condensing flow channel, the condensing and filtering module is detachably configured in the integrated passageway module to receive the gas comprising hydrogen from the integrated passageway module and filter the gas comprising hydrogen in the condensing flow channel.

18. The hydrogen gas generating system of claim 16, wherein the hydrogen gas generating device further comprises a nebulizer coupled to the integrated passageway module to receive the humidified gas comprising hydrogen from the integrated passageway module, the nebulizer is configured to generate an atomized gas and mix the atomized gas with the gas comprising hydrogen to form and output a health gas.
